# EUROPÄISCHE PATENTSCHRIFT

(11) **EP 0 885 200 B1**
(45) Veröffentlichungstag und Bekanntmachung des Hinweises auf die Patenterteilung: **21.05.2008**
(21) Anmeldenummer: 97906098.5
(22) Anmeldetag: 20.02.1997
(51) Int. Cl.: C07D 251/16, C07D 251/22, C07D 251/46, C07D 239/42, C07D 239/46, A01N 47/36

(54) **SUBSTITUIERTE ARYLSULFONYL(THIO)HARNSTOFFE ALS HERBIZIDE**
SUBSTITUTED ARYL SULPHONYL(THIO)UREAS USED AS HERBICIDES
ARYLSULFONYL(THIO)UREES SUBSTITUEES UTILISEES COMME HERBICIDES

(30) Priorität: 05.03.1996 DE 19608445
(43) Veröffentlichungstag der Anmeldung: 23.12.1998
(60) Teilanmeldung: 06009626.0
(73) Patentinhaber: Bayer CropScience AG, 40789 Monheim (DE)
(72) Erfinder: GESING, Ernst, Rudolf, F., D-40699 Erkrath (DE); KIRSTEN, Rolf, D-40789 Monheim (DE); KLUTH, Joachim, D-40764 Langenfeld (DE); MÜLLER, Klaus-Helmut, D-40593 Düsseldorf (DE); DREWES, Mark, Wilhelm, D-40764 Langenfeld (DE); JANSEN, Johannes, Rudolf, D-40789 Monheim (DE); PHILIPP, Ulrich, 50674 Köln (DE); RIEBEL, Hans-Jochem, D-42113 Wuppertal (DE); SCHALLNER, Otto, D-40789 Monheim (DE); DOLLINGER, Markus, D-51381 Leverkusen (DE); SANTEL, Hans-Joachim, D-51371 Leverkusen (DE)
(74) Vertreter: Schwenk, Norbert
(86) Internationale Anmeldenummer: PCT/EP1997/000798
(87) Internationale Veröffentlichungsnummer: WO 1997/032861

(56) Entgegenhaltungen:
- EP-A- 0 023 422
- EP-A- 0 044 807
- EP-A- 0 096 002
- CHEMICAL ABSTRACTS, vol. 124, no. 15, 9.April 1996 Columbus, Ohio, US; abstract no. 196428, SUNG, NACK-DO ET AL: "Herbicidal activity and persistency in aqueous solution of ortho- disubstituted benzenesulfonylurea derivatives" XP002031533 & HAN'GUK NONGHWA HAKHOECHI (1995), 38(6), 570-6 CODEN: JKACA7;ISSN: 0368-2897, 1995,

## Beschreibung

Die Erfindung betrifft neue substituierte Arylsulfonyl(thio)harnstoffe, Verfahren zu ihrer Herstellung und ihre Verwendung als Herbizide

Es ist bereits bekannt, dass bestimmte substituierte Sulfonylharnstoffe herbizide Eigenschaften aufweisen (vgl. DE 2715786, EP 1514, EP 23422). Dokument EP-A-0044 807 offenbart N-Phenylsulfonyl-N-Triazinylharnstoffe mit herbiziden und wuchsregulierenden Eigenschaften. Die ausgeführten und nicht nur generisch offenbarten N-Phenylsulfonyl-N-Triazinylharnstoffe zeichnen sich dadurch aus, dass der Phenylrest ein 2,6-Disubstitutionsmuster aufweist. Die herbizide Wirksamkeit und die Verträglichkeit gegenüber Kulturpflanzen dieser Verbindungen sind jedoch nicht in allen Belangen zufriedenstellend.

Es wurden nun die neuen substituierten Arylsulfonyl harnstoffe der allgemeinen Formel (I) 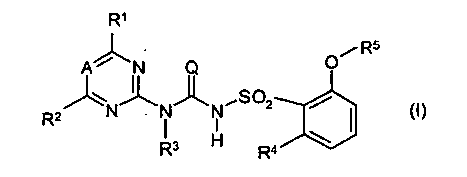 in welcher
- A: für Stickstoff steht,
- Q: für Sauerstoff steht,
- R¹: für Halogen, für gegebenenfalls durch Halogen substituiertes Alkoxy mit 1 bis 4 Kohlenstoffatomen in den Alkylgruppen, für Alkyl oder Dialkylamino mit jeweils 1 bis 4 Kohlenstoffatomen in den Alkylgruppen steht,
- R²: für gegebenenfalls durch Halogen substituiertes Alkoxy mit 1 bis 4 Kohlenstoffatomen in den Alkylgruppen oder für Alkyl, Alkylthio oder Dialkylamino mit jeweils 1 bis 4 Kohlenstoffatomen in den Alkylgruppen steht,
- R³: für Wasserstoff steht,
- R⁴: für jeweils gegebenenfalls durch Halogen substituiertes Alkyl oder unsubstituiertes Alkoxy mit jeweils 1 bis 6 Kohlenstoffatomen in den Alkylgruppen
Oder für Cycloalkyloxy mit jeweils 3 bis 6 Kohlenstoffatomen in den Cycloalkylgruppen steht,
- R⁵: für gegebenenfalls durch Halogen substituiertes Alkyl mit 1 bis 6 Kohlenstoffatomen in den Alkylgruppen oder für Cycloalkyl mit 3 bis 6 Kohlenstoffatomen in den Cycloalkylgruppen,
sowie die Natriumsalze von Verbindungen der Formel (I).

Man erhält die neuen substituierten Arylsulfonylharnstoffe der allgemeinen Formel (I), wenn man
(a) Aminoazine der allgemeinen Formel (II) 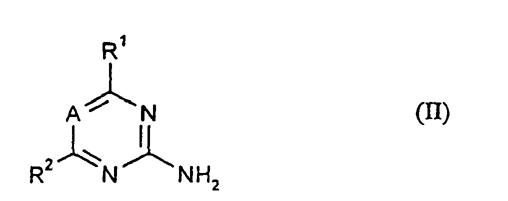 in welcher
   A, R¹ und R² die oben angegebene Bedeutung haben,
   mit Arylsulfonylisocyanaten der allgemeinen Formel (III) 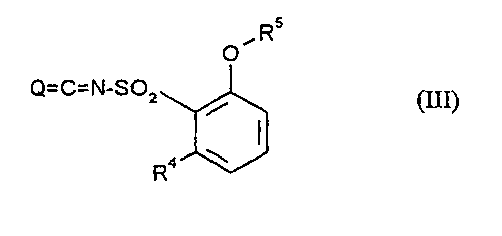 in welcher
   Q, R⁴ und R⁵ die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(b) substituierte Aminoazine der allgemeinen Formel (IV) 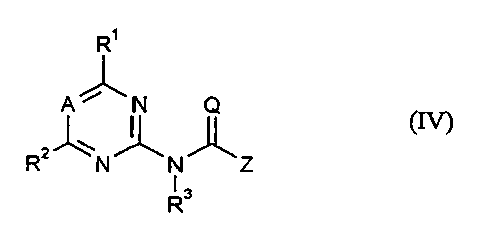 in welcher
   A, Q, R¹ und R² die oben angegebene Bedeutung haben,
   Z für Halogen, Alkoxy oder Aryloxy steht und
   R³ die oben angegebene Bedeutung hat oder für die Gruppierung -C(Q)-Z steht,
   mit Arensulfonamiden der allgemeinen Formel (V) 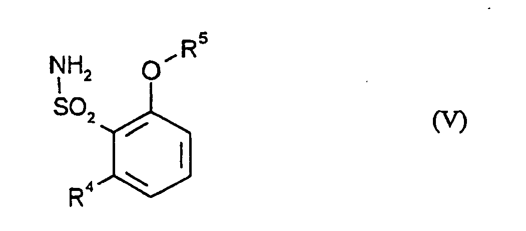 in welcher
   R⁴ und R⁵ die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(c) Aminoazine der allgemeinen Formel (II) 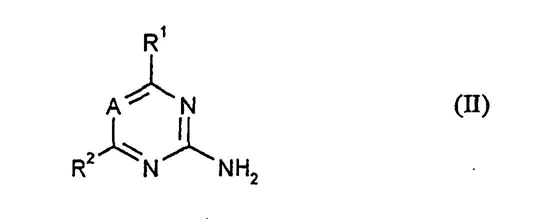 in welcher
   A, R¹ und R² die oben angegebene Bedeutung haben,
   mit substituierten Arensulfonamiden der allgemeinen Formel (VI) 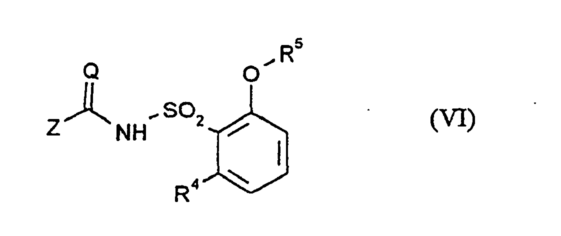 in welcher
   Q, R⁴ und R⁵ die oben angegebene Bedeutung haben und
   Z für Halogen, Alkoxy oder Aryloxy steht,
   gegebenenfalls in Gegenwart eines Reaktionshilfsmittels und gegebenenfalls in Gegenwart eines Verdünnungsmittels umsetzt,
   und gegebenenfalls die nach den Verfahren (a), (b) und (c) erhaltenen Verbindungen der Formel (I) nach üblichen Methoden in Salze überführt.

Die neuen substituierten Arylsulfonylharnstoffe der allgemeinen Formel (I) zeichnen sich durch starke herbizide Wirksamkeit aus.

Gegenstand der Erfindung sind weiter Natrium-, -Salze von Verbindungen der Formel (I), in welcher A, Q, R¹, R², R³, R⁴ und R⁵ die oben vorzugsweise angegebenen Bedeutungen haben.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Beispiele für die erfindungsgemäßen Verbindungen der Formel (I) sind in den nachstehenden Gruppen aufgeführt.

### Gruppe 1

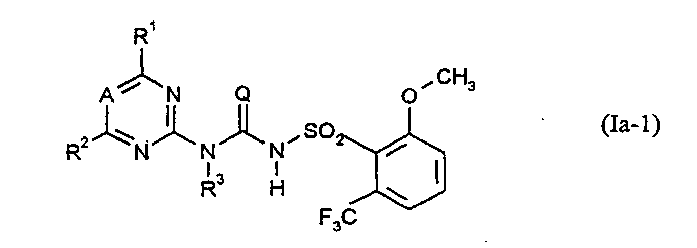

A, Q, R¹, R² und R³ haben darin beispielhaft die nachstehend aufgeführte Bedeutung:

| A | Q | R¹ | R² | R³ |
|---|---|---|---|---|
| N | O | CH₃ | OCH₃ | H |
| N | O | OCH₃ | OCH₃ | H |
| N | O | CH₃ | CH₃ | H |
| N | O | OCHF₂ | N(CH₃)₂ | H |
| N | O | CH₃ | SCH₃ | H |
| N | O | C₂H₅ | OCH₃ | H |
| N | O | CH₃ | OC₂H₅ | H |
| N | O | OCH₃ | 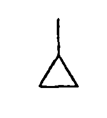 | H |
| N | O | 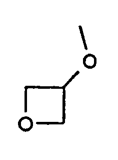 | 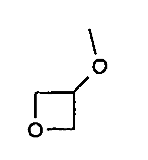 | H |
| N | O | CH₃ | 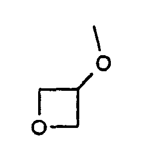 | H |
| N | S | CH₃ | OCH₃ | H |

### Gruppe 2

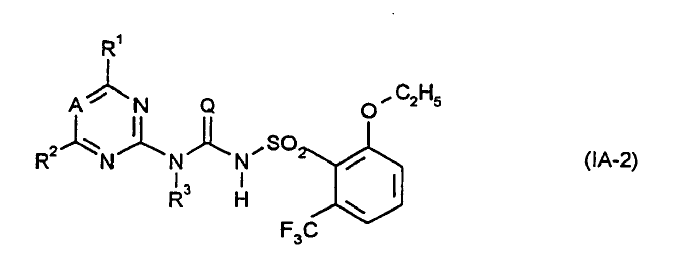

A, Q, R¹, R² und R³ haben hierbei beispielhaft die oben in Gruppe 1 angegebene Bedeutung.

### Gruppe 3

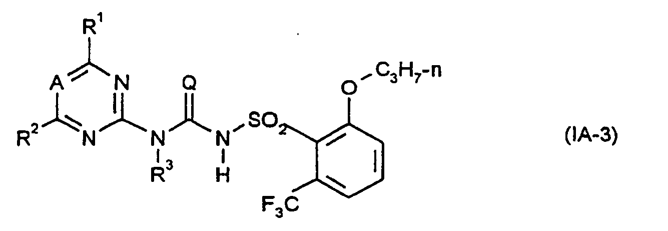

A, Q, R¹, R² und R³ haben hierbei beispielhaft die oben in Gruppe 1 angegebene Bedeutung.

### Gruppe 4

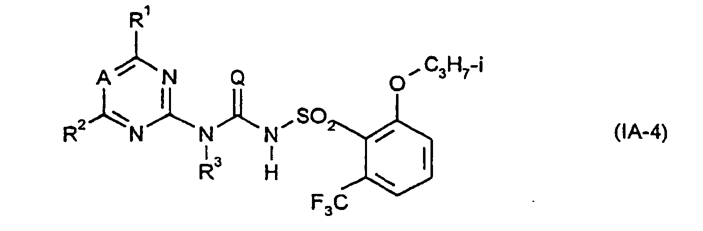

A, Q, R¹, R² und R³ haben hierbei beispielhaft die oben in Gruppe 1 angegebene Bedeutung.

### Gruppe 5

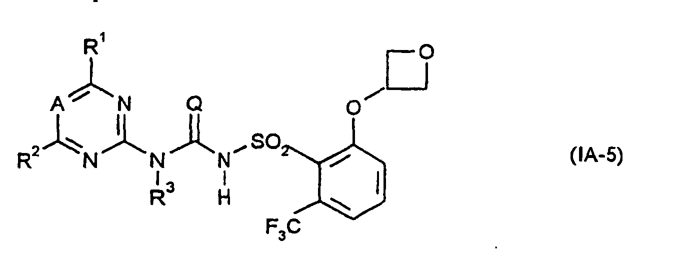

A, Q, R¹, R² und R³ haben hierbei beispielhaft die oben in Gruppe 1 angegebene Bedeutung.

### Gruppe 6

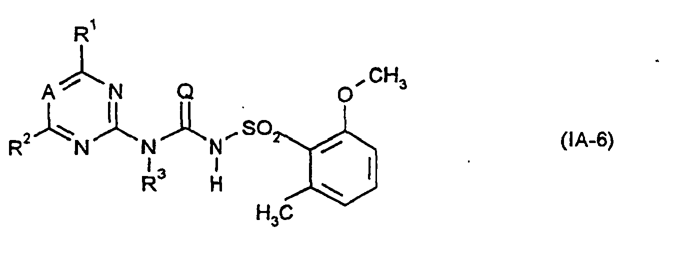

A, Q, R¹, R² und R³ haben hierbei beispielhaft die oben in Gruppe 1 angegebene Bedeutung.

### Gruppe 7

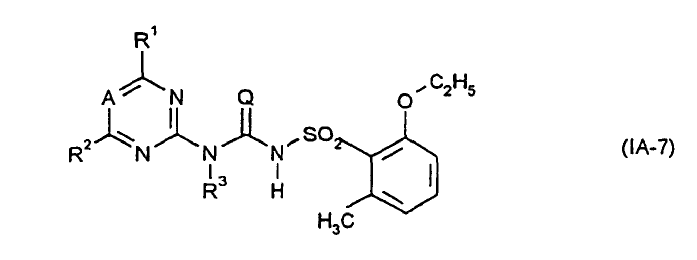

A, Q, R¹, R² und R³ haben hierbei beispielhaft die oben in Gruppe 1 angegebene Bedeutung.

### Gruppe 8

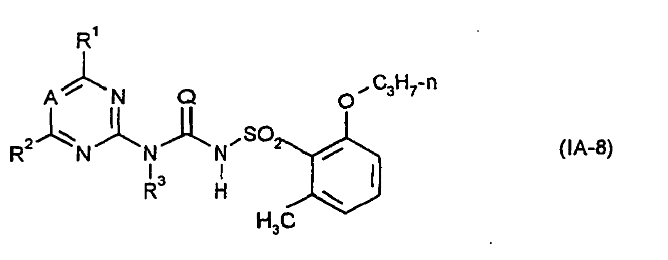

A, Q, R¹, R² und R³ haben hierbei beispielhaft die oben in Gruppe 1 angegebene Bedeutung.

### Gruppe 9

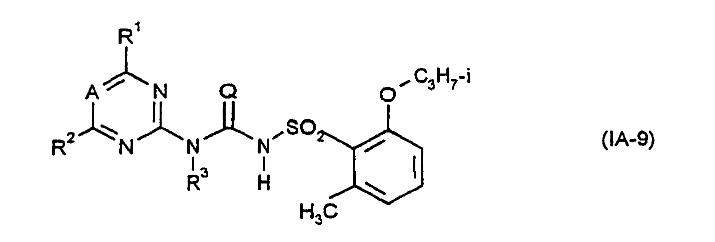

A, Q, R¹, R² und R³ haben hierbei beispielhaft die oben in Gruppe 1 angegebene Bedeutung.

### Gruppe 10

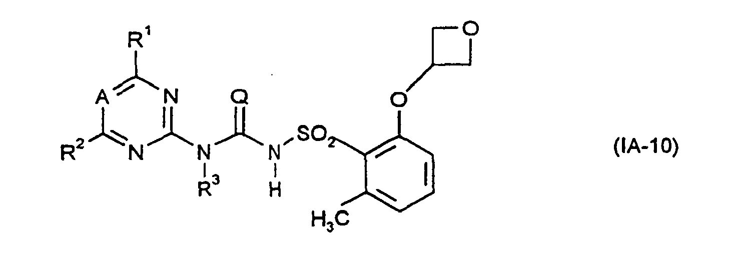

A, Q, R¹, R² und R³ haben hierbei beispielhaft die oben in Gruppe 1 angegebene Bedeutung.

### Gruppe 11

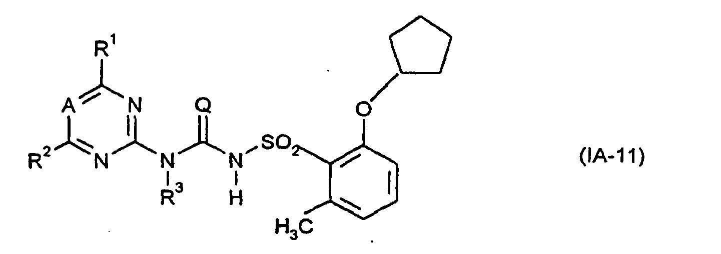

A, Q, R¹, R² und R³ haben hierbei beispielhaft die oben in Gruppe 1 angegebene Bedeutung.

### Gruppe 12

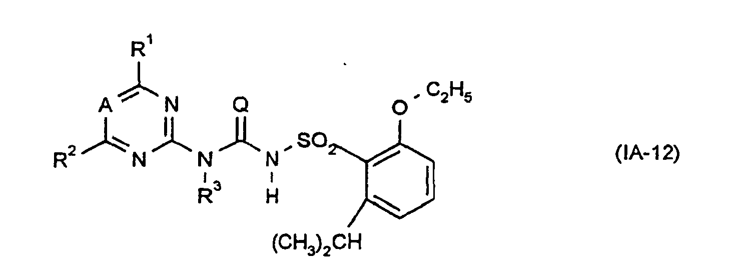

A, Q, R¹, R² und R³ haben hierbei beispielhaft die oben in Gruppe 1 angegebene Bedeutung.

### Gruppe 13

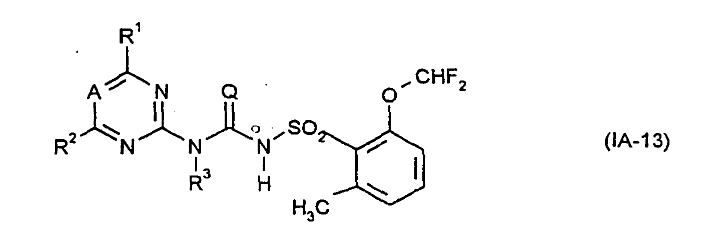

A, Q, R¹, R² und R³ haben hierbei beispielhaft die oben in Gruppe 1 angegebene Bedeutung.

### Gruppe 14

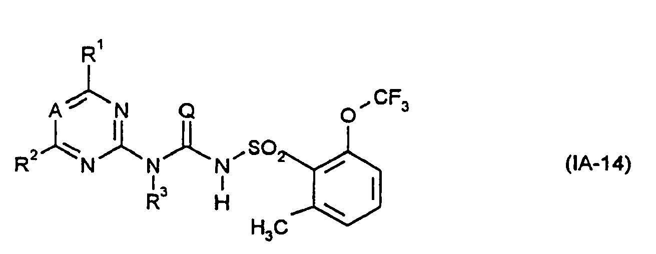

A, Q, R¹, R² und R³ haben hierbei beispielhaft die oben in Gruppe 1 angegebene Bedeutung.

Verwendet man beispielsweise 2-Amino-4-methoxy-6-methyl-pyrimidin und 2-Ethoxy-6-trifluormethyl-phenylsulfonylisocyanat als Ausgangsstoffe, so kann der Reaktionsablauf beim erfindungsgemäßen Verfahren (a) durch das folgende Formelschema skizziert werden:

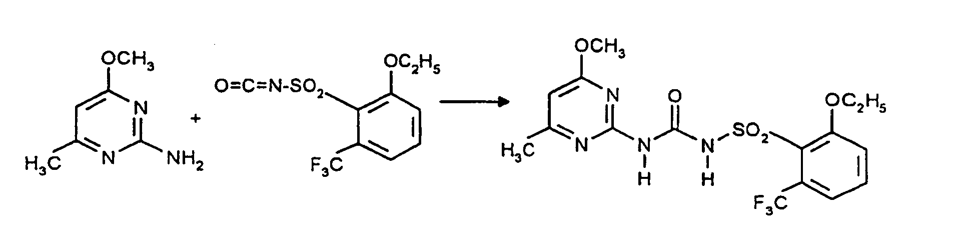

Verwendet man beispielsweise 2-Methoxycarbonylamino-4-methoxy-6-trifluormethyl-1,3,5-triazin und 2-Methyl-6-trifluormethoxy-benzolsulfonamid als Ausgangsstoffe, so kann der Reaktionsablauf beim erfindungsgemäßen Verfahren (b) durch das folgende Formelschema skizziert werden:

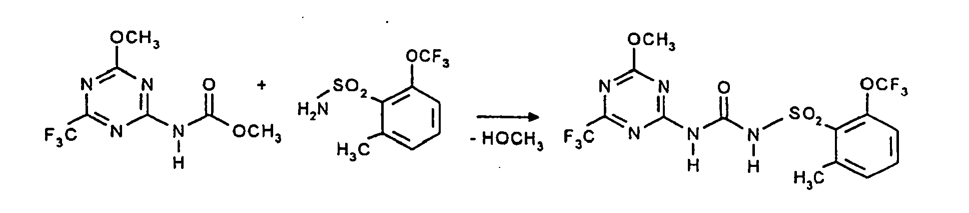

Verwendet man beispielsweise 2-Amino-4-chlor-6-methoxy-pyrimidin und N-(2,6-Dimethoxy-phenylsulfonyl)-O-phenyl-urethan als Ausgangsstoffe, so kann der Reaktionsablauf beim erfindungsgemäßen Verfahren (c) durch das folgende Formelschema skizziert werden:

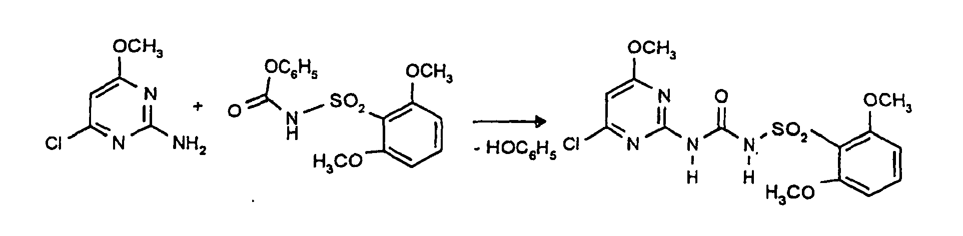

Die bei den erfindungsgemäßen Verfahren (a) und (c) zur Herstellung der Verbindungen der allgemeinen Formel (I) als Ausgangsstoffe zu verwendenden Aminoazine sind durch die Formel (II) allgemein definiert. In der Formel (II) haben A, R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, R¹ und R² angegeben wurden.

Die Aminoazine der Formel (II) sind bekannte, zum Teil im Handel erhältliche Synthesechemikalien.

Die beim erfindungsgemäßen Verfahren (a) weiter als Ausgangsstoffe zu verwendenden Arylsulfonylisocyanate sind durch die Formel (III) allgemein definiert. In der Formel (III) haben Q, R⁴ und R⁵ vorzugsweise bzw. insbesondere diejenigen Bedeutungen, die bereits oben im Zusammenhang mit der Beschreibung der Verbindungen der Formel (I) für Q, R⁴ und R⁵ angegeben wurden.

Die Ausgangsstoffe der Formel (III) sind bekannt und/oder können nach an sich bekannten Verfahren hergestellt werden (vgl. DE 3208189, EP 23422, EP 64322, EP 44807, EP 216504, Herstellungsbeispiele).

Man erhält die Arylsulfonyliso cyanate der Formel (III), wenn man Arensulfonamide der allgemeinen Formel (V) - oben - mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie z.B. Diazabicyclo[2.2.2]-octan, und in Gegenwart eines Verdünnungsmittels, wie z.B. Toluol, Xylol oder Chlorbenzol, bei Temperaturen zwischen 80°C und 150°C umsetzt und nach Ende der Umsetzung die flüchtigen Komponenten unter vermindertem Druck abdestilliert.

Die beim erfindungsgemäßen Verfahren (b) zur Herstellung der Verbindungen der Formel (I) als Ausgangsstoffe zu verwendenden substituierten Aminoazine sind durch die Formel (IV) allgemein definiert. In der Formel (IV) haben A, Q, R¹ und R² diejenigen Bedeutungen, die bereits oben im Zusammenhang mit der Beschreibung der Verbindungen der Formel (I) für A, Q, R¹ bzw. R² angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (IV) sind bekannt und/oder können nach an sich bekannten Verfahren hergestellt werden (vgl. US 4690707, DE 19501174, Herstellungsbeispiele).

Die beim erfindungsgemäßen Verfahren (b) weiter als Ausgangsstoffe zu verwendenden Arensulfonamide sind durch die Formel (V) allgemein definiert. In der Formel (V) haben R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R⁴, und R⁵ angegeben wurden.

Die Ausgangsstoffe der Formel (V) sind bekannt und/oder können nach an sich bekannten Verfahren hergestellt werden (vgl. DE 3208189, EP 23422, EP 64322, EP 44807, EP 216504, DE 19525162, Herstellungsbeispiele).

Die beim erfindungsgemäßen Verfahren (c) zur Herstellung der Verbindungen der Formel (I) als Ausgangsstoffe zu verwendenden substituierten Arensulfonamide sind durch die Formel (VI) allgemein definiert. In der Formel (VI) haben Q, R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q, R⁴ bzw. R⁵ angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (VI) sind bekannt und/oder können nach an sich bekannten Verfahren hergestellt werden (vgl. die Herstellungsbeispiele).

Als Verdünnungsmittel zur Durchführung der erfindungsgemäßen Verfahren (a), (b) und (c) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methyl-formanilid, N-Methyl-pyrrolidon oder Hexamethylphosphorsäuretriamid; Ester, wie Essigsäuremethylester oder Essigsäureethylester; Sulfoxide, wie Dimethylsulfoxid; Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die erfindungsgemäßen Verfahren (a), (b) und (c) werden vorzugsweise in Gegenwart eines geeigneten Reaktionshilfsmittels durchgeführt. Als solche kommen alle üblichen anorganischen oder organischen Basen in Frage. Hierzu gehören beispielsweise Erdalkali- oder Alkalimetall- -hydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a), (b) und (c) in einem größeren Bereich variiert werden. Im allgemeinen arbeitet man bei Temperaturen zwischen -20°C und +150°C, vorzugsweise zwischen 0°C und +120°C.

Die erfindungsgemäßen Verfahren (a), (b) und (c) werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren (a), (b) und (c) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur Bekämpfung von monokotylen und dikotylen Unkräutern sowohl im Vorauflaufals auch im Nachauflauf-Verfahren. Sie zeigen starke herbizide Aktivität und ein breites Wirkungsspektrum bei Anwendung auf den Boden und auf oberirdische Pflanzenteile.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide in Frage, beispielsweise Anilide, wie z.B. Diflufenican und Propanil; Arylcarbonsäuren, wie z.B. Dichlorpicolin-säure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

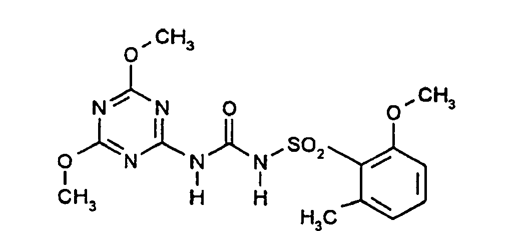

### (Verfahren (b))

Eine Mischung aus 2,0 g (10 mMol) 2-Methoxy-6-methyl-benzolsulfonamid, 4,0 g (10 mMol) N,N-Bis-phenoxycarbonyl-2-amino-4,6-dimethoxy-1,3,5-triazin, 1,1 g (10 mMol) Kalium-t-butanolat und 50 ml Acetonitril wird 15 Stunden bei ca. 20°C gerührt. Dann wird im Wasserstrahlvakuum eingeengt und der Rückstand mit 50 ml IN-Salzsäure und 50 ml Methylenchlorid verrührt. Die organische Phase wird abgetrennt, mit Wasser gewaschen, mit Natriumsulfat getrocknet und filtriert. Das Filtrat wird im Wasserstrahlvakuum eingeengt, der Rückstand mit i-Propanol digeriert und das kristallin angefallene Produkt durch Absaugen isoliert.

Man erhält 2,1 g (55% der Theorie) N-(4,6-Dimethoxy-1,3,5-triazin-2-yl)-N'-(2-methoxy-6-methyl-phenylsulfonyl)-harnstoff vom Schmelzpunkt 163°C.

Analog zu Herstellungsbeispiel 1 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

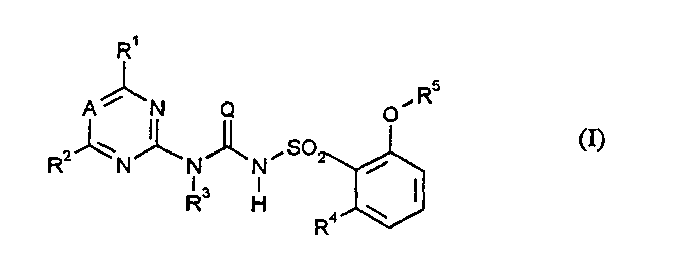

**Tabelle 1: Beispiele für die Verbindungen der Formel (I)**

| Bsp.- Nr. | A | Q | R¹ | R² | R³ | R⁴ | R⁵ | Schmelzpunkt (°C) |
|---|---|---|---|---|---|---|---|---|
| 4 | N | O | CH₃ | OCH₃ | H | CF₃ | C₂H₅ | 115 |
| 5 | N | O | CH₃ | OCH₃ | H | CF₃ | CH₃ | 214 |
| 6 | N | O | OCH₃ | OCH₃ | H | CF₃ | CH₃ | 189 |
| 7 | N | O | OCH₃ | OCH₃ | H | CF₃ | C₂H₅ | 176 |
| 9 | N | O | CH₃ | OCH₃ | H | CF₃ | C₃H₇-n | 142 |
| 10 | N | O | OCH₃ | OCH₃ | H | CF₃ | C₃H₇-n | 162 |
| 12 | N | O | CH₃ | OCH₃ | H | CF₃ | C₃H₇-i | 168 |
| 13 | N | O | OCH₃ | OCH₃ | H | CF₃ | C₃H₇-i | 200 |
| 18 | N | O | N(CH₃)₂ | OCH₂CF₃ | H | CF₃ | CH₃ | 222 |
| 19 | N | O | N(CH₃)₂ | OCH₂CF₃ | H | CF₃ | C₂H₅ | 209 |
| 20 | N | O | N(CH₃)₂ | OCH₂CF₃ | H | CF₃ | C₃H₇-n | 175 |
| 21 | N | O | N(CH₃)₂ | OCH₂CF₃ | H | CF₃ | C₃H₇-i | 204 |
| 31 | N | O | OCH₃ | OCH₃ | H | CH₃ | C₂H₅ | 181 |
| 32 | N | O | CH₃ | OCH₃ | H | CH₃ | C₂H₅ | 170 |
| 38 | N | O | OCH₃ | OCH₃ | H | CH₃ | C₃H₇-i | 191 |
| 39 | N | O | OCH₃ | OCH₃ | H | CH₃ | C₃H₇-i | 205 |
| 40 | N | O | CH₃ | OCH₃ | H | CH₃ | CH₃ | 193 |
| 45 | N | O | N(CH₃)₂ | OCH₂CF₃ | H | CH₃ | C₂H₅ | 187 |
| 48 | N | O | OCH₃ | OCH₃ | H | CH₃ | C₃H₇-n | 156 |
| 49 | N | O | CH₃ | OCH₃ | H | CH₃ | C₃H₇-n | 150 |
| 51 | N | O | OCH₃ | OCH₃ | H | C₃H₇-i | C₂H₅ | 157 |
| 52 | N | O | N(CH₃)₂ | OCH₂CF₃ | H | CH₃ | CH₃ | 214 |
| 53 | N | O | N(CH₃)₂ | OCH₂CF₃ | H | CH₃ | C₃H₇-i | 202 |
| 55 | N | O | OCH₃ | OCH₃ | H | CH₃ | 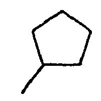 | 198 |
| 56 | N | O | CH₃ | OCH₃ | H | CH₃ | 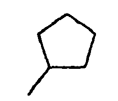 | 183 |
| 57 | N | O | OCH₃ | OCH₃ | H | CH₃ | C₄H₉-s | 174 |
| 64 | N | O | CH₃ | OCH₃ | H | C₃H₇-i | CH₃ | 136 |
| 66 | N | O | CH₃ | OCH₃ | H | CH₃ | CHF₂ | 197 |
| 67 | N | O | OCH₃ | OCH₃ | H | OC₃H₇-n | C₃H₇-n | 140 |
| 69 | N | O | CH₃ | OCH₃ | H | OCH₃ | C₃H₇-i | 152 |
| 70 | N | O | CH₃ | OCH₃ | H | OC₄H₉-n | C₄H₉-n | 90 |
| 71 | N | O | CH₃ | OCH₃ | H | OC₃H₇-i | C₃H₇-i | 80 |
| 72 | N | O | CH₃ | OCH₃ | H | OC₂H₅ | C₃H₇-i | 118 |
| 73 | N | O | CH₃ | OCH₃ | Na | OCH₃ | CH₃ | 203 |
| 77 | N | O | CH₃ | CH₃ | H | CF₃ | CH₃ | 155 |
| 78 | N | O | CH₃ | SCH₃ | H | CF₃ | CH₃ | 178 |
| 79 | N | O | CH₃ | N(CH₃)₂ | H | CF₃ | CH₃ | 213 |
| 80 | N | O | CH₃ | OC₂H₅ | H | CF₃ | CH₃ | 121 |
| 81 | N | O | C₂H₅ | OCH₃ | H | CF₃ | CH₃ | 117 |
| 82 | N | O | OCH₂CF₂CHF₂ | N(CH₃)₂ | H | CF₃ | CH₃ | 185 |
| 83 | N | O | CH₃ | OCH₃ | Na | C₃H₇-i | CH₃ | 170 |
| 84 | N | O | OCH₃ | OCH₃ | H | C₃H₇-i | C₃H₇-n | 149 |
| 85 | N | O | OCH₃ | OCH₃ | H | C₃H₇-i | CH₃ | 187 |
| 86 | N | O | CH₃ | OCH₃ | H | C₃H₇-i | C₂H₅ | 163 |
| 90 | N | O | CH₃ | OCH₃ | Na | CH₃ | CH₃ | 149 |
| 91 | N | O | CH₃ | CH₃ | H | 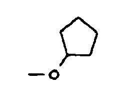 | 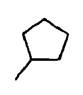 | 125 |
| 92 | N | O | CH₃ | OCH₃ | Na | OC₃H₇-i | C₃H₇-i | 178 |
| 93 | N | O | Cl | OCH₃ | Na | OCH₃ | C₃H₇-i | 172 |
| 94 | N | O | CH₃ | OCH₃ | Na | OC₂Hₛ | C₂H₅ | 145 |
| 95²⁾ | N | O | CH₃ | OCH₃ | Na | OCH₃ | C₃H₇-n | NMR-Daten |
| 96 | N | O | OCH₃ | OCH₃ | H | OCH₃ | C₂H₅ | 110 |
| 97 | N | O | OCH₃ | OCH₃ | H | OC₂H₅ | C₂H₅ | 142 |
| 98 | N | O | OCH₃ | OCH₃ | H | OCH₃ | C₃H₇-n | 188 |
| 99 | N | O | OCH₃ | OCH₃ | H | OC₂H₅ | C₄H₉-s | 136 |
| 105 | N | O | CH₃ | OCH₃ | H | OC₂H₅ | C₃H₇-n | 110 |
| 106 | N | O | CH₃ | OCH₃ | H | OCH₃ | CH₃ | 177 |
| 107 | N | O | CH₃ | OCH₃ | H | OCH₃ | 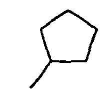 | 166 |
| 108 | N | O | CH₃ | OCH₃ | H | OC₂H₅ | C₄H₉-i | 85 |
| 109 | N | O | CH₃ | OCH₃ | H | OCH₃ | C₂H₅ | 148 |
| 110 | N | O | CH₃ | OCH₃ | H | OCH₃ | C₄H₉-n | 125 |
| 111 | N | O | CH₃ | OCH₃ | H | OC₂H₅ | 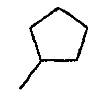 | 130 |
| 113 | N | O | CH₃ | OCH₃ | H | CH₃ | CF₃ | 178 |
| 115 | N | O | CH₃ | OCH₃ | H | C₂H₅ | CF₃ | 140 |
| 117 | N | O | CH₃ | OCH₃ | H | CH₃ | CF₂CHF₂ | 184 |
| 119 | N | O | CH₃ | OCH₃ | H | CH₃ | CH₂CF₃ | 178 |
| 121 | N | O | CH₃ | OCH₃ | H | C₂H₅ | CHF₂ | 157 |
| 123 | N | O | CH₃ | OCH₃ | H | C₃H₇-n | CF₃ | 77 |

| | | | | | | | | |
|---|---|---|---|---|---|---|---|---|
| 2) NMR-Daten zu Beispiel 95: ¹H-NMR (300MHz; D₂O): δ = 0,96 (t, CH₃); 1,77 (m, O-CH₂-CH₂-CH₃); 2,42 (s, CH₃); 3,87 (s, OCH₃); 3,98 (s, OCH₃); 4,04 (t, O-CH₂-); 6,79 (br.d, 2 aromat. H); 7,48 (br.t, 1 aromat. H) ppm. | | | | | | | | |

### Ausgangsstoffe der Formel (III):

### Beispiel (III-1)

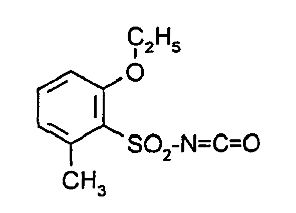

21,5 g (0,1 Mol) 2-Ethoxy-6-methyl-benzolsulfonsäureamid und 10 g (0,1 Mol) n-Butylisocyanat werden in 100 ml Chlorbenzol zum Sieden erhitzt. Bei Rückflußtemperatur wird 4 Stunden Phosgen eingeleitet. Die klare Lösung wird unter vermindertem Druck eingeengt und der Rückstand feindestilliert. Bei einem Druck von 0,8 mbar und einer Kopftemperatur von 135 - 140°C geht 2-Ethoxy-6-methylphenylsulfonylisocyanat über, das in der Vorlage erstarrt.

Man erhält 7,9 g 2-Ethoxy-6-methyl-phenylsulfonylisocyanat als farbloses Produkt vom Schmelzpunkt 40°C.

### Ausgangsstoffe der Formel (IV):

### Beispiel (IV-1)

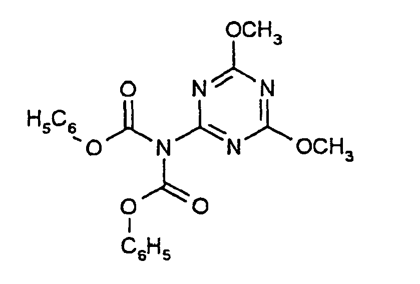

70 g (0,44 Mol) Chlorameisensäure-phenylester werden tropfenweise unter Rühren zu einer Mischung aus 31 g (0,20 Mol) 2-Amino-4,6-dimethoxy-s-triazin und 100 ml Pyridin gegeben. Die Reaktionsmischung wird ca. 15 Stunden bei 20°C bis 25°C gerührt und anschließend im Wasserstrahlvakuum eingeengt. Der Rückstand wird in Wasser aufgenommen und dann mit konz. Salzsäure angesäuert. Das hierbei kristallin angefallene Produkt wird durch Absaugen isoliert. Man erhält 74,2 g (91% der Theorie) 2-(N,N-Bis-phenoxycarbonyl-amino)-4,6-dimethoxy-s-triazin vom Schmelzpunkt 125°C.

### Ausgangsstoffe der Formel (V):

### Beispiel (V-1)

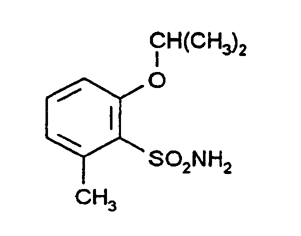

64,6 g (0,26 Mol) 2-Isopropoxy-6-methyl-benzolsulfochlorid werden in 350 ml 25%iger wässriger Ammoniaklösung 12 Stunden bei 20°C gerührt. Das kristallin angefallene Produkt wird anschließend durch Absaugen isoliert.

Man erhält 54 g (90% der Theorie) 2-Isopropoxy-6-methyl-benzolsulfonamid vom Schmelzpunkt 78°C.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung gespritzt. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:

| | | |
|---|---|---|
| 0 % | = | keine Wirkung (wie unbehandelte Kontrolle) |
| 100 % | = | totale Vernichtung |

In diesem Test zeigen beispielsweise die Verbindungen gemäß Herstellungsbeispiel 1, 4, 5, 6, 7, 9, 10, 12, 13, 31, 32, 38, 39, 40, 48, 49, 56, und 66 bei Aufwandmengen zwischen 30 g und 125 g a.i. pro Hektar sehr starke Wirkung gegen Unkräuter.
"a.i." = "active ingredient" = Wirkstoff

**Tabelle A: Pre-emergence-Test/Gewächshaus**

| Wirkstoff gemäß Herstellungsbeispiel Nr. | Aufwandmenge (g ai./ha) | Alopecurus | Lolium | Sorghum | Amaranthus | Chenopodium | Steltaria |
|---|---|---|---|---|---|---|---|
| 4 | 60 | 100 | 100 | 100 | 100 | 100 | 95 |
| 5 | 60 | 100 | 100 | 100 | 100 | 100 | 100 |
| 6 | 60 | 100 | 100 | 100 | 100 | 100 | 100 |
| 7 | 60 | 100 | 95 | 100 | 95 | 100 | 95 |
| 1 | 60 | 100 | 100 | 100 | 100 | 100 | 100 |
| 9 | 30 | 100 | 100 | 90 | 100 | 100 | 100 |
| 10 | 60 | 100 | 100 | 100 | 100 | 100 | 100 |
| 12 | 30 | 100 | 100 | 100 | 100 | 95 | 100 |
| 13 | 60 | 100 | 100 | 100 | 100 | 100 | 100 |
| 31 | 60 | 95 | 100 | 90 | 95 | 100 | 95 |
| 32 | 60 | 95 | 100 | 95 | 100 | 100 | 100 |
| 38 | 30 | 100 | 100 | 100 | 80 | 100 | 100 |
| 39 | 30 | 100 | 100 | 100 | 100 | 100 | 100 |
| 40 | 30 | 100 | 100 | 100 | 100 | 100 | 100 |
| 48 | 60 | 100 | 100 | 100 | 100 | 100 | 100 |
| 49 | 30 | 100 | 100 | 100 | 100 | 100 | 100 |
| 56 | 60 | 100 | 100 | 90 | 100 | 100 | 100 |
| 66 | 60 | 95 | 95 | 95 | 95 | 95 | 95 |

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle. Es bedeuten:
0 % =keine Wirkung (wie unbehandelte Kontrolle)
100 % =totale Vernichtung

In diesem Test zeigen beispielsweise die Verbindungen gemäß Herstellungsbeispiel 4, 5, 6, 7, 9, 12, 31, 32, 38, 39, 40, 48, 49, 51, und 66 sehr starke Wirkung gegen Unkräuter.

**Tabelle B: Post emergence-Test/Gewächshaus**

| Wirkstoff gemäß Herstellungsbiespiel-Nr. | Aufwandmenge (gai./ha) | Alopecurus | Echinochloa | Abutilon | Matricaria |
|---|---|---|---|---|---|
| 4 | 30 | 90 | 90 | 100 | 100 |
| 5 | 30 | 80 | 100 | 100 | 100 |
| 6 | 30 | 60 | 100 | 90 | 100 |
| 7 | 30 | 95 | 95 | 95 | 100 |
| 9 | 8 | 90 | 80 | 100 | 95 |
| 12 | 30 | 95 | 90 | 95 | 100 |
| 31 | 60 | 80 | 80 | 100 | 100 |
| 32 | 60 | 70 | 90 | 100 | 100 |
| 38 | 30 | 60 | 70 | 95 | 95 |
| 39 | 30 | 90 | 95 | 100 | 100 |
| 40 | 30 | 100 | 95 | 95 | 100 |
| 48 | 60 | 95 | 70 | 100 | 90 |
| 49 | 30 | 90 | 80 | 100 | 100 |
| 51 | 60 | 80 | 90 | 100 | 90 |
| 66 | 60 | 80 | 80 | 100 | 100 |

## Patentansprüche

1. Substituierte Arylsulfony()harnstoffe der allgemeinen Formel (I) 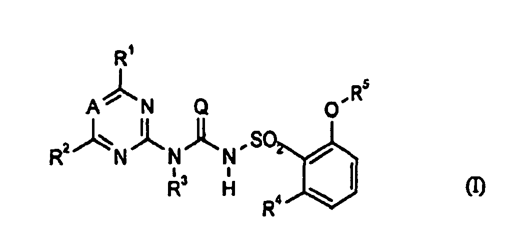 in welcher
A für Stickstoff steht,
Q für Sauerstoff steht,
R¹ für Halogen, für gegebenenfalls durch Halogen substituiertes Alkoxy mit 1 bis 4 Kohlenstoffatomen in den Alkylgruppen, für Alkyl oder Dialkylamino mit jeweils 1 bis 4 Kohlenstoffatomen in den Alkylgruppen steht,
R² für gegebenenfalls durch Halogen substituiertes Alkoxy mit 1 bis 4 Kohlenstoffatomen in den Alkylgruppen oder für Alkyl, Alkylthio oder Dialkylamino mit jeweils 1 bis 4 Kohlenstoffatomen in den Alkyl-gruppen steht,
R³ für Wasserstoff steht,
R⁴ für jeweils gegebenenfalls durch Halogen substituiertes Alkyl oder unsubstituiertes Alkoxy mit jeweils 1 bis 6 Kohlenstoffatomen in den Alkylgruppen oder für cycloalkyloxy mit jeweils 3 bis 6 Kohlenstoffatomen in den Cycloalkylgruppen steht,
R⁵ für gegebenenfalls durch Halogen substituiertes Alkyl mit 1 bis 6 Kohlenstoffatomen in den Alkylgruppen oder für Cycloalkyl mit 3 bis 6 Kohlenstoffatomen in den Cycloalkylgruppen,
sowie die Natrium-Salze von Verbindungen der Formel (I).

2. Verfahren zur Herstellung von Verbindungen der Formel (I) gemäß Anspruch 1, **dadurch gekennzeichnet, dass** man
(a) Aminoazine der allgemeinen Formel (II) 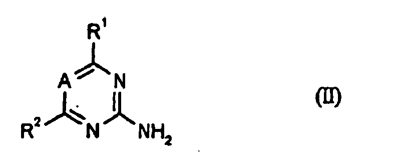 in welcher
A, R¹ und R² die in Anspruch 1 angegebene Bedeutung haben,
mit Arylsulfonyliso(thio)cyanaten der allgemeinen Formel (III) 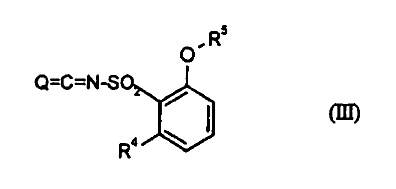 in welcher
Q, R⁴ und R⁵ die in Anspruch 1 angegebene Bedeutung haben,
gegebenenfalls in Gegenwart eines Realnionshilfsmittels und gegebenenfalls in Gegenwart eines Verdünnungsmittels umsetzt,
oder dass man
(b) substituierte Aminoazine der allgemeinen Formel (IV) 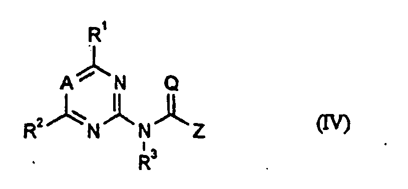 in welcher
A, Q, R¹ und R² die oben angegebene Bedeutung haben,
Z für Halogen, Alkoxy oder Aryloxy steht und
R³ die in Anspruch 1 angegebene Bedeutung hat oder für die Gruppierung - C(Q)-Z steht,
mit Arensulfonamiden der allgemeinen Formel (V) 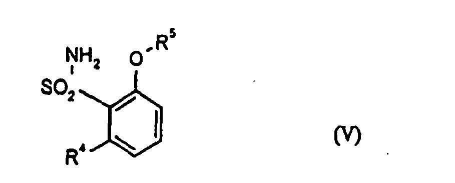 in welcher
R⁴ und R⁵ die oben angegebene Bedeutung haben,
gegebenenfalls in Gegenwart eines Reaktionshilfsmittels und gegebenenfalls in Gegenwart eines Verdünnungsmittels umsetzt,
oder dass man
(c) Aminoazine der allgemeinen Formel (II) 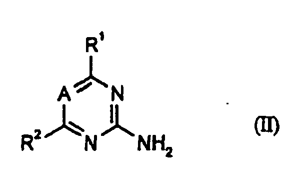 in welcher
A, R¹ und R² die oben angegebene Bedeutung haben,
mit substituierten Arensulfonamiden der allgemeinen Formel (VI) 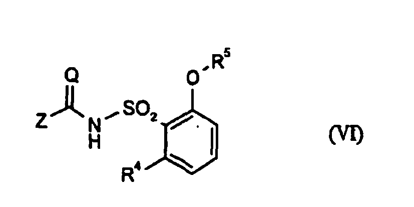 in welcher
Q, R⁴ und R⁵ die oben angegebene Bedeutung haben und
Z für Halogen, Alkoxy oder Aryloxy steht,
gegebenenfalls in Gegenwart eines Reaktionshilfsmittels und gegebenenfalls in Gegenwart eines Verdünnungsmittels umsetzt,
und gegebenenfalls die nach den Verfahren (a), (b) und (c) erhaltenen Verbindungen der Formel (I) nach üblichen Methoden in Salze überführt.

3. Herbizide Mittel, **gekennzeichnet durch** einen Gehalt an mindestens einer Verbindung der Formel (I) gemäß Anspruch 1.

4. Verwendung von Verbindungen der allgemeinen Formel (I) gemäß Anspruch 1 zur Bekämpfung von unerwünschtem Pflanzenwachstum.

5. Verfahren zur Bekämpfung von Unkräutern, **dadurch gekennzeichnet, dass** man Verbindungen der allgemeinen Formel (I) gemäß Anspruch 1 auf die Unkräuter oder ihren Lebensraum einwirken lässt.

6. Verfahren zur Herstellung von herbiziden Mitteln, **dadurch gekennzeichnet, dass** man Verbindungen der allgemeinen Formel (I) gemäß Anspruch 1 mit Streckmitteln und/oder oberflächenaktiven Mitteln vermischt.

## Claims

1. Substituted arylsulphonyl ureas of the general formula (I) 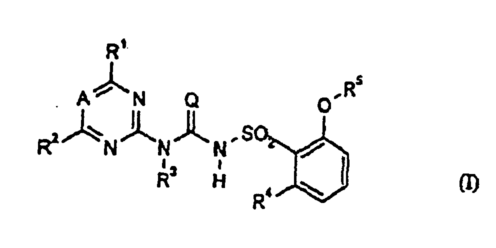 in which
A represents nitrogen,
Q represents oxygen,
R¹ represents, halogen, represents optionally halogen-substituted alkoxy having 1 to 4 carbon atoms in the alkyl groups, represents alkyl or dialkylamino having in each case 1 to 4 carbon atoms in the alkyl groups,
R² represents optionally halogen-substituted alkoxy having 1 to 4 carbon atoms in the alkyl groups, or represents alkyl, alkylthio or dialkylamino having in each case 1 to 4 carbon atoms in the alkyl groups,
R³ represents hydrogen,
R⁴ represents in each case optionally halogen-substituted alkyl or unsubstituted alkoxy having in each case 1 to 6 carbon atoms in the alkyl groups, or represents cycloalkyloxy having in each case 3 to 6 carbon atoms in the cycloalkyl groups,
R⁵ represents optionally halogen-substituted alkyl having in each case 1 to 6 carbon atoms in the alkyl groups, or represents cycloalkyl having in each case 3 to 6 carbon atoms in the cycloalkyl groups,
and the sodium salts of compounds of the formula (I) .

2. Process for preparing compounds of the formula (I) according to Claim 1, **characterized in that**
(a)aminoazines of the general formula (II) 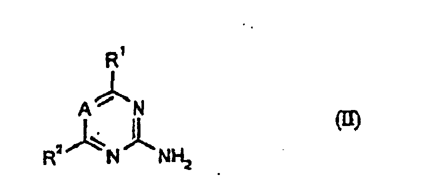 in which
A, R¹ and R² are each as defined in Claim 1,
are reacted with arylsulphonyl iso(thio)cyanates of the general formula (III) 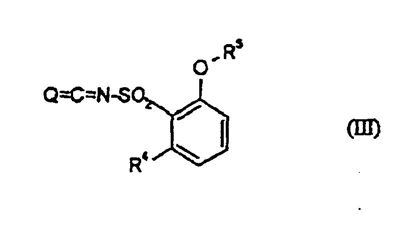 in which
Q, R⁴ and R⁵ are each as defined in Claim 1,
if appropriate in the presence of a reaction auxiliary and if appropriate in the presence of a diluent,
or
(b) substituted aminoazines of the general formula (IV) 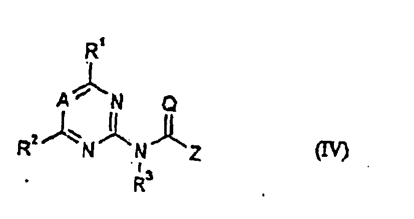 in which
A, Q, R¹ and R² are each as defined above,
Z represents halogen, alkoxy or aryloxy and
R³ is as defined in Claim 1 or represents the grouping -C(Q)-Z
are reacted with arenesulphonamides of the general formula (V) 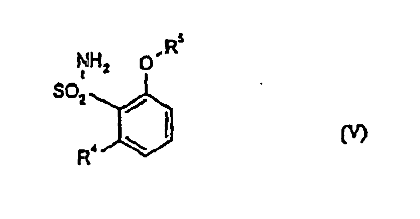 in which
R⁴ and R⁵ are each as defined above,
if appropriate in the presence of a reaction auxiliary and if appropriate in the presence of a diluent,
or
(c) aminoazines of the general formula (II) 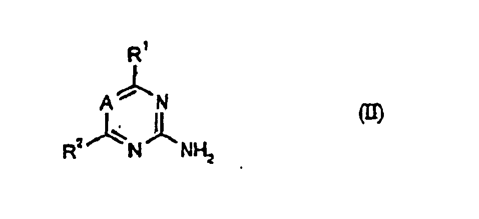 in which
A, R¹ and R² are each as defined above,
are reacted with substituted arenesulphonamides of the general formula (VI) 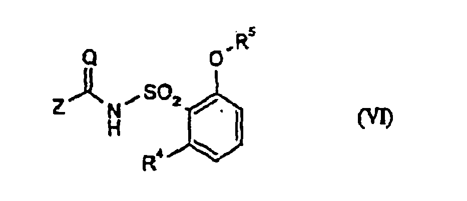 in which
Q, R⁴ and R⁵ are each as defined above and
Z represents halogen, alkoxy or aryloxy,
if appropriate in the presence of a reaction auxiliary and if appropriate in the presence of a diluent,
and the compounds of the formula (I) obtained by processes (a), (b) and (c) are, if appropriate, converted into salts by customary methods.

3. Herbicidal compositions, **characterized in that** they contain at least one compound of the formula (I) according to Claim 1.

4. The use of compounds of the general formula (I) according to Claim 1 for controlling undesirable plant growth.

5. Method for controlling weeds, **characterized in that** compounds of the general formula (I) according to Claim 1 are allowed to act on the weeds or their habitat.

6. Process for preparing herbicidal compositions, **characterized in that** compounds of the general formula (I) according to Claim 1 are mixed with extenders and/or surfactants.

## Revendications

1. Arylsulfonylurées substituées de formule générale (I) 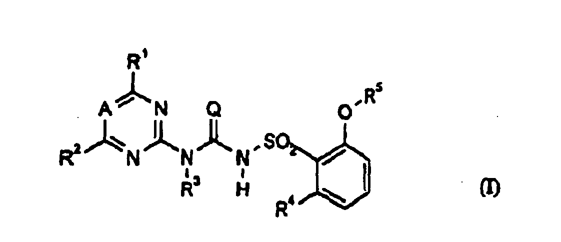 dans laquelle
A représente un atome d'azote,
Q représente un atome d'oxygène,
R¹ représente un halogène, un alcoxy éventuellement substitué par un halogène avec 1 à 4 atomes de carbone dans les groupes alkyle, un alkyle ou dialkylamino avec respectivement 1 à 4 atomes de carbone dans les groupes alkyle,
R² représente un alcoxy éventuellement substitué par un halogène avec 1 à 4 atomes de carbone dans les groupes alkyle ou un alkyle, alkylthio ou dialkylamino avec respectivement 1 à 4 atomes de carbone dans les groupes alkyle,
R³ représente un atome d'hydrogène,
R⁴ représente un alkyle éventuellement substitué par un halogène ou un alcoxy non substitué avec respectivement 1 à 6 atomes de carbone dans les groupes alkyle,
ou un cycloalkyloxy avec respectivement 3 à 6 atomes de carbone dans les groupes cycloalkyle,
R⁵ représente un alkyle éventuellement substitué par un halogène avec 1 à 6 atomes de carbone dans les groupes alkyle ou un cycloalkyle avec 3 à 6 atomes de carbone dans les groupes cycloalkyle,
et les sels de sodium des composés de formule (I).

2. Procédé de préparation de composés de formule (I) selon la revendication 1, **caractérisé en ce que** l'on met à réagir
(a) une aminoazine de formule générale (II) 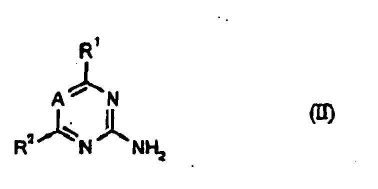 dans laquelle
A, R¹ et R² ont la signification indiquée dans la revendication 1,
avec des arylsulfonyliso(thio)cyanates de formule générale (III) 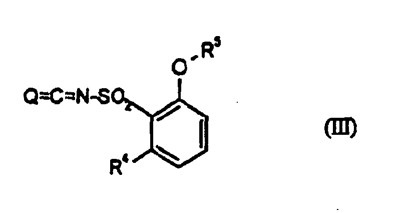 dans laquelle
Q, R⁴ et R⁵ ont la signification indiquée dans la revendication 1,
éventuellement en présence d'un adjuvant de réaction et éventuellement en présence d'un agent diluant,
ou **en ce que** l'on met à réagir
(b) une aminoazine substituée de formule générale (IV) 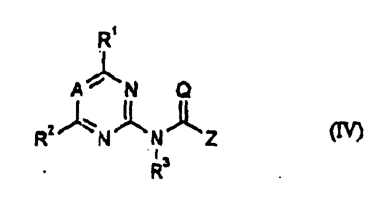 dans laquelle
A, Q, R¹ et R² ont la signification indiquée ci-dessus, Z représente un halogène, alcoxy ou aryloxy et
R³ a la signification indiquée dans la revendication 1 ou représente le groupement -C(Q)-Z,
avec des arènesulfonamides de formule générale (V) 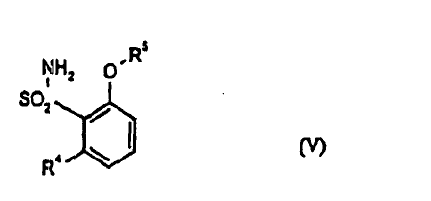 dans laquelle
R⁴ et R⁵ on la signification indiquée ci-dessus, éventuellement en présence d'un adjuvant de réaction et éventuellement en présence d'un agent diluant,
on **en ce que** l'on met à réagir
(c) une aminoazine de formule générale (II) 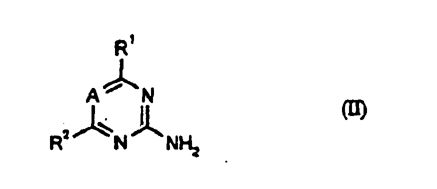 dans laquelle
A, R¹ et R² ont la signification indiquée ci-dessus,
avec des arènesulfonamides substitués de formule générale (VI) 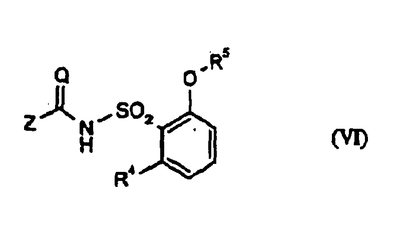 dans laquelle
Q, R⁴ et R⁵ ont la signification indiquée ci-dessus et Z représente un halogène, alcoxy ou aryloxy,
éventuellement en présence d'un adjuvant de réaction et éventuellement en présence d'un agent diluant,
et on transforme éventuellement en sels les composés de formule (I) obtenus selon les procédés (a), (b) et (c) selon des procédés usuels.

3. Agents herbicides, **caractérisés par** une teneur en au moins un composé de formule (I) selon la revendication 1.

4. Utilisation de composés de formule générale (I) selon la revendication 1 pour la lutte contre la croissance de plantes non souhaitée.

5. Procédé de lutte contre les mauvaises herbes, **caractérisé en ce que** l'on fait agir des composés de formule générale (I) selon la revendication 1 sur les mauvaises herbes ou leur espace vital.

6. Procédé de préparation d'agents herbicides, **caractérisé en ce que** l'on mélange des composés de formule générale (I) selon la revendication 1 avec des diluants et/ou des agents tensio-actifs.
